**Europäisches Patentamt**

(19) **European Patent Office**

**Office européen des brevets**

(11) Publication number : **0 459 463 B1**

(12) # EUROPEAN PATENT SPECIFICATION

(45) Date of publication of patent specification :
**17.08.94 Bulletin 94/33**

(51) Int. Cl.⁵ : **C07C 21/18, C07C 17/00**

(21) Application number : **91108824.3**

(22) Date of filing : **29.05.91**

(54) **Process for preparing 1-chloro-1,2,2-trifluoroethylene or 1,2,2-trifluoroethylene.**

(30) Priority : **31.05.90 JP 143053/90**
**31.05.90 JP 143054/90**
**11.09.90 JP 241691/90**
**11.09.90 JP 241692/90**

(43) Date of publication of application :
**04.12.91 Bulletin 91/49**

(45) Publication of the grant of the patent :
**17.08.94 Bulletin 94/33**

(84) Designated Contracting States :
**DE FR GB**

(56) References cited :
**EP-A- 0 355 907**
**DE-B- 1 044 800**
**JP-A- 438 454**

(73) Proprietor : **DAIKIN INDUSTRIES, LIMITED**
**Umeda Center Building,**
**4-12 Nakazaki-nishi 2-chome,**
**Kita-ku**
**Osaka-shi, Osaka-fu 530 (JP)**

(72) Inventor : **Ichikawa, Masaru, c/o Hokkaido**
**Univ-Caralysis**
**Research Center,**
**Nishi 10-Chome**
**Kita 11-jo**
**Kita-ku, Sapporo-shi, Hokkaido (JP)**
Inventor : **Ohnishi, Ryuichiro c/o Hokkaido**
**Univ.Caralysis**
**Research Center**
**Nishi 10-chome**
**Kita 11-jo**
**Kita-ku Sapporo-shi Hokkaido (JP)**
Inventor : **Suzuki, Hisao c/o Hokkaido**
**Univ.Caralysis**
**Research Center**
**Nishi 10-chome**
**Kita 11-jo**
**Kita-ku Sapporo-shi Hokkaido (JP)**

(74) Representative : **Hansen, Bernd, Dr.**
**Dipl.-Chem. et al**
**Hoffmann, Eitle & Partner**
**Patent- und Rechtsanwälte,**
**Postfach 81 04 20**
**D-81904 München (DE)**

EP 0 459 463 B1

## Description

The present invention relates to a process for preparing 1-chloro-1,2,2-trifluoroethylene or 1,2,2-trifluoroethylene in high selectivities and yields, which product is useful as a raw material for the preparation of polychlorotrifluoroethylene and substituted fluorohydrocarbons.

Japanese Patent Publication No. 8454/1968 discloses a process for preparing 1,2,2-trifluoroethylene (hereinafter referred to as "3FH") by reacting 1,1,2-trichloro-1,2,2-trifluoroethane (hereinafter referred to as "R-113") and hydrogen at a temperature of 200 to 300°C in the presence of a catalyst comprising palladium supported on activated carbon (Pd/carbon). By this process, 1-chloro-1,2,2-trifluoroethylene (hereinafter referred to as "3FCL") is obtained together with 3FH, but selectivities of other by-products are high and therefore the selectivity of 3FH and 3FCL is only 40 to 85 %.

By a process preparing a mixture of 3FH and 3FCL from R-113 and hydrogen in the presence of a platinum group metal supported on alkali magnesium fluoride as a catalyst which is disclosed in EP-A-053 657, 3FH and 3FCL are obtained at a high selectivity of 90 % or higher. But, the reaction temperature should be as high as 500°C. By the reaction at a low temperature of 200 to 300°C in the presence of the above catalyst containing 0.5 % of palladium, the selectivity is still low.

For the preparation of 3FCL, various other processes are also known. An example is a non-catalytic process comprising dechlorination of R-113 with zinc in a liquid phase (cf. Japanese Patent Publication Nos. 45322/1972, 5207/1982 and 5208/1982), and another is a catalytic process comprising dechlorination of R-113 with hydrogen in a gas phase (cf. Japanese Patent Publication No. 26484/1972, Japanese Patent Kokai Publication Nos. 185734/ 1985, 61936/1987 and 29328/1989 and GB Patent No. 698,386).

By the liquid phase non-catalytic process using zinc, the yield is comparatively high, but treatment of by-produced zinc chloride is troublesome. In addition, the liquid phase process should use an anhydrous organic solvent. By the gas phase dechlorination, the satisfactory high selectivity is achieved only under reduced pressure (for example, a selectivity of 95 % under 41,3x10$^3$Pa (310 Torr) in Japanese Patent Kokai Publication No. 61936/1987 but at a high reaction temperature (for example, a selectivity of 82 to 95 % at 500°C in EP-A-053 657). The catalyst life is limited due to the serious coke-poisoning.

EP-A-0 355 907 discloses a process for the hydrogenation of 1,1,2-trichloro-1,2,2-trifluoroethane in chlorotrifluoroethylene and trifluoroethylene in the presence of molecular hydrogen. Said hydrogenation reaction is catalysed by a catalytic composition comprising a porous oxygenated support or a support based on carbon and a metal of the group VIII of the periodic system and one or more compounds selected from alkali or alkaline earth salts.

One object of the present invention is to provide a process for preparing 3FH or 3FCL at a comparatively low temperature under atmospheric pressure.

Another object of the present invention is to provide a process for preparing 3FH and 3FCL, by which the ratio of prepared 3FH and 3FCL can be controlled.

According to the present invention, there is provided a process for preparing 3FCL and 3FH comprising reacting R-113 and hydrogen in the presence of a catalyst which comprises at least one metal selected from the group consisting of palladium, rhodium and ruthenium and at least one metal selected from the group consisting of mercury, lead, cadmium, tin, indium, copper, bismuth, thallium and silver and a carrier selected from the group consisting of $Al_2O_3$, $SiO_2$ and activated carbon.

The reactions which proceed in the process of the present invention are as follows:

$$CCl_2FCF_2Cl + H_2 \rightarrow CClF{=}CF_2 + 2HCl$$
$$CCl_2FCF_2Cl + 2H_2 \rightarrow CFH{=}CF_2 + 3HCl.$$

In the present invention, the catalyst comprises at least one metal selected from the group consisting of palladium, rhodium and ruthenium (basic metals) and at least one metal selected from the group consisting of mercury, lead, cadmium, tin, indium, copper, bismuth, thallium and silver (additive metals), and a carrier selected from the group consisting of $Al_2O_3$, $SiO_2$ and activated carbon.

An amount of the basic metal to be supported on the carrier is from 0.1 to 10 % by weight, preferably from 0.5 to 5 % by weight.

The metal can be deposed on the carrier by a per se conventional method. For example, the carrier material is dipped in a solution of a salt or a metal compound of the metal such as a nitrate, a carbonate, a sulfate, a halide (eg. a chloride, a fluoride), a hydroxide, a phosphate, a perchlorate, an organic metal compound or a salt with an organic acid (e.g. acetates, acetylacetonates, carbonyls) Then, the solvent is removed, and the residue is subjected to calcination and reduction with hydrogen at 27°C to 500°C (300 to 773 K.)

Two kinds of metals are simultaneously deposed on the carrier, although they may be successively deposed on the carrier.

The molar ratio of the additive metal to the basic metal may vary according to the kinds of the two netals, the intended selectivities of 3FCL and 3FH, for instance. The molar ratio of the additive metal to the basic metal is usually from 0.01:1 to 10:1, preferably from 0.2:1 to 4:1.

In case of bismuth, this molar ratio is at least 0.05:1 preferably at least 0.2:1, and in case or the metals other than bismuth, this molar ratio is at least 0.2:1, preferably at least 0.5:1. When this molar ratio is too large, the conversion decreases. Therefore, the maximum molar ratio is preferably 4:1.

For example, when a Hg/Pd ratio is less than 0.5, the selectivity of 3FH greatly decreases so that the overall selectivity of the olefins is decreased. When a Tl/Pd ratio is 4, 3FCL is obtained at a selectivity of 96 % or higher. When the Tl/Pd ratio is 0.5, 3FH is predominantly produced.

According to the present invention, by the selection of the additive metal, the molar ratio of 3FCL to 3FH in the product can be changed. For example, when mercury, lead and copper are used as the additive metal, the molar ratio of 3FCL to 3FH is 0.11, 0.58 and 5.8, respectively.

A molar ratio of hydrogen to R-113 is preferably from 0.5 to 4.0, more preferably from 0.5 to 3.5. If this ratio is large, hydrogenated paraffin compounds are hardly produced and the high selectivity of 3FCL and 3FH is maintained. However, a molar ratio of larger than 4.0 is uneconomical. When the molar ratio is smaller than 0.5, the conversion decreases.

A W/F ratio corresponding to a contact time, in which W is an amount (g) of the catalyst and is a total flow rate (ml/sec.) under the normal state, is in a range of between 0.6 and 5.8. In this range, the selectivity is not influenced substantially, but the conversion is influenced. When this ratio is smaller than 0.6, the conversion is unacceptably small.

The reaction temperature is from 150 to 400°C, preferably from 200 to 350°C. In the temperature range between 200°C and 350°C, 3FCL and 3FH are produced at a high selectivity of 87 to 100 %.

The present invention will be illustrated by the following Examples.

General procedures for supporting an additive metal on a carrier (I)

A suitable amount of a salt of an additive metal to be supported was dissolved in water (30 ml). Into this solution there were added formalin (0.2 g) and a pellet form palladium catalyst of 3.2 mm in diameter and 3.2 mm in height comprising 0.5 % of palladium carried on $Al_2O_3$ (5 g) (Catalyst A); a catalyst prepared by pulverizing said pellet form palladium catalyst to 60 mesh or lower (4 g) (Catalyst B); a powdery catalyst comprising 2 % of palladium carried on activated carbon (1.5 g) (Catalyst C); or a powdery catalyst comprising 5 % of a basic metal carried on $Al_2O_3$ (4 g) (Catalyst D). All of the catalysts are commercially available from N. E. CHEMICAL CATALYST. Then, the mixture was aged at 50°C for 2 to 3 hours. An amount of the additive metal was adjusted to obtain the intended molar ratio of the additive metal to the basic metal.

Then, water was removed by a rotary evaporator and the residue was dried in air at 100°C for 12 hours.

Each of the produced Catalysts A, B and C was pretreated in a hydrogen stream at a temperature of 300 to 400°C for 2 hours. When bismuth was used, the catalyst was pretreated in an oxygen stream at 300°C for 2 hours.

Example 1

$HgCl_2$, as the additive metal salt, was used in such an amount that the molar ratio of Hg to Pd in the catalyst was 4:1.

In a Hastelloy C made tube reactor having an inner diameter of 20 mm, Catalyst A (4 g) modified with $HgCl_2$ as above was charged, and through the reactor tube, a mixture of R-113 and hydrogen in a molar ratio of 1:2 was flowed at a total flow rate of 21 ml/min. at 200°C.

The conversion was 17.2 %, and the selectivities of 3FCL and 3FH were 24.6 % and 75.4 %, respectively.

Example 2

In the same manner as in Example 1 but using $Pb(NO_3)_2$ as the additive metal salt, adjusting the Pb/Pd molar ratio to 4:1 and keeping the reaction temperature at 280°C, the reaction was carried out.

The conversion was 46.8 %, and the selectivities of 3FCL, 3FH, R-123a ($CHClFCClF_2$), R-141a ($CHCl_2CH_2F$), R-160 ($CH_2ClCH_3$) and R-142 ($CHF_2CH_2Cl$) were 23.4 %, 71.0 %, 2.7 %, 1.1 %, 0.6 % and 0.3 %, respectively.

Example 3

In the same manner as in Example 1 but using $CdCl_2$ as the additive metal salt, adjusting the Cd/Pd molar ratio to 4 :1 and keeping the reaction temperature at 280°C, the reaction was carried out.

The conversion was 21.6 %, and the selectivities of 3FCL, 3FH, R-160, R-123a, R-142 and R-141a were 33.4 %, 58.8 %, 2.0 %, 1.9 %, 1.6 % and 1.5 %, respectively.

Example 4

In the same manner as in Example 1 but using $SnCl_2$ as the additive metal salt, adjusting the Sn/Pd molar ratio to 4:1 and keeping the reaction temperature at 250°C, the reaction was carried out.

The conversion was 15.9 %, and the selectivities of 3FCL, 3FH and R-142 were 79.3 %, 15.3 % and 2.5 %, respectively.

Example 5

In the same manner as in Example 1 but using $InCl_3$ as the additive metal salt, adjusting the In/Pd molar ratio to 4:1 and keeping the reaction temperature at 250°C, the reaction was carried out.

The conversion was 10.4 %, and the selectivities of 3FCL, 3FH, R-142, R-160, R-123a and R-141a were 71.9 %, 16.1 %, 4.6 %, 2.9 %, 2.3 % and 1.3 %, respectively.

## Example 6

In the same manner as in Example 1 but using CuC1$_2$ as the additive metal salt, adjusting the Cu/Pd molar ratio to 4:1 and keeping the reaction temperature at 250°C, the reaction was carried out.

The conversion was 18.7 %, and the selectivities of 3FCL, 3FH and R-142 were 79.5 %, 17.2 % and 1.9 %, respectively.

## Example 7

AgNO$_3$, as the additive metal salt, was used in such an amount that the molar ratio of Ag to Pd in the catalyst was 4:1.

In a stainless steel made tube reactor having an inner diameter of 7.2 mm, Catalyst B (1.3 g) modified with AgNO$_3$ as above was charged, and through the reactor tube, a mixture of R-113 and hydrogen in a molar ratio of 1:2 was flowed at a total flow rate of 27 ml/min. at 250°C.

The conversion was 32.2 %, and the selectivities of 3FH, 3FCL, R-142, R-123a, R-160 and R-141a were 29.3 %, 67.0 %, 1.2 %, 0.9 %, 0.5 % and 0.3 %, respectively.

## Example 8

In the same manner as in Example 7 but using HgCl$_2$ as the additive metal salt and flowing a mixture of hydrogen and R-113 at a total flow rate of 60 ml/min., the reaction was carried out.

The conversion was 54.7 %, and the selectivities of 3FCL, 3FH, R-152a (CF$_2$HCH$_3$), R-123a and R-142 were 9.3 %, 87.4 %, 2.2 %, 0.7 % and 0.4 %, respectively.

## Example 9

In the same manner as in Example 8 but flowing the mixture of hydrogen and R-113 at a total flow rate of 27 ml/min., the reaction was carried out.

The conversion was 45.2 %, and the selectivities of 3FH, 3FCL, R-123a, R-152a, R-142 and R-160 were 82.7 %, 7.6 %, 4.8 %, 1.5 %, 0.6 % and 0.5 %, respectively.

## Example 10

In the same manner as in Example 8 but flowing a mixture of R-113 and hydrogen in a molar ratio of 1:4, the reaction was carried out.

The conversion was 40.0 %, and the selectivities of 3FH, 3FCL, R-123a, R-141a, R-152a, R-142 and R-160 were 76.9 %, 12.0 %, 5.8 %, 2.1 %, 1.7 %, 0.7 and 0.5 %, respectively.

## Example 11

In the same manner as in Example 7 but using Pb(NO$_3$)$_2$ as the additive metal salt, adjusting the Pb/Pd molar ratio to 4:1 and flowing a mixture of R-113 and hydrogen in a molar ratio of 1:1 at a total flow rate of 18 ml/min., the reaction was carried out.

The conversion was 11.2 %, and the selectivities of 3FH, 3FCL, R-123a, R-152a and R-142 were 60.4 %, 35.1 %, 2.1 %, 1.0 % and 0.9 %, respectively.

## Example 12

In the same manner as in Example 11 but using a mixture of R-113 and hydrogen in a molar ratio of 1:4, the reaction was carried out.

The conversion was 12.0 %, and the selectivities of 3FH and 3FCL were 56.3 % and 43.7 %, respectively.

## Example 13

In the same manner as in Example 12 but keeping the reaction temperature at 300°C, the reaction was carried out.

The conversion was 22.9 %, and the selectivities of 3FH, 3FCL, R-123a, R-142 and R-152a were 44.3 %, 49.4 %, 1.9 %, 1.4 % and 1.4 %, respectively.

## Example 14

In the same manner as in Example 9 but using Catalyst D (1.3 g) comprising palladium as the base metal and modified with HgCl$_2$ in the Hg/Pd molar ratio of 1:1, the reaction was carried out.

The conversion was 60.8 %, and the selectivities of 3FCL, 3FH, R-123a, R-134a (CF$_3$CFH$_2$), R-141a, R-142 and R-160 were 79.0 %, 12.1 %, 3.6 %, 2.4 %, 1.5 %, 0.6 % and 0.5 %, respectively.

## Example 15

In the same manner as in Example 8 but using Catalyst C (0.6 g) modified with the said amount of HgCl$_2$ and keeping the reaction temperature at 280°C, the reaction was carried out.

The conversion was 63.9 %, and the selectivities of 3FH, 3FCL and R-123a were 16.0 %, 62.2 % and 21.9 %, respectively.

## Example 16

In the same manner as in Example 1 but using Catalyst C (0.6 g) modified with Pb(NO$_3$)$_2$ as the additive metal salt in the Pb/Pd molar ratio of 4:1 and keeping the reaction temperature at 280°C, the reaction was carried out.

The conversion was 28.5 %, and the selectivities of 3FH, 3FCL and R-123a were 6.0 %, 69.2 % and 24.8 %, respectively.

Comparative Example 1

In the same manner as in Example 8 but using no metal other than palladium, the reaction was carried out.

The conversion was 64.3 %, and the selectivities of 3FCL, 3FH, R-143, R-141a, R-123a, R-142, R-160 and R-152a were 0 %, 23.0 %, 40.2 %, 18.7 %, 7.0 %, 6.0 %, 4.6 % and 0.7 %, respectively.

Comparative Example 2

In the same manner as in Examples 1 to 7 but using no metal other than palladium and keeping the reaction temperature at 150°C, the reaction was carried out.

The conversion was 24.7 %, and the selectivities of 3FH, R-143, R-141a, R-160, R-142 and R-123a were 1.5 %, 44.2 %, 41.2 %, 1.0 %, 3.7 % and 8.5 %, respectively.

Example 17

As the additive metal salt, BiCl$_3$ was used in such an amount that the molar ratio of Bi to Pd in the catalyst was 0.4:1.

In a glass tube reactor having an inner diameter of 10 mm, Catalyst D comprising palladium as the base metal and modified with BiCl$_3$ as above was charged, and through the reactor tube, a mixture of R-113 and hydrogen in a molar ratio of 1:3 was flowed at a total flow rate of 31 ml/min. at 250°C.

The conversion was 96.4 %, and the selectivities of 3FH, 3FCL, R-152a and R-123a were 81.3 %, 8.9 %, 6.1 % and 1.3 %, respectively.

Example 18

In a glass tube reactor having an inner diameter of 10 mm, Catalyst D (1 g) comprising ruthenium as the base metal and modified with TlNO$_3$ in the molar ratio of Tl/Rh of 2:1 was charged, and through the reactor tube, a mixture of R-113 and hydrogen in a molar ratio of 3:4 was flowed at a total flow rate of 35 ml/min. at 230°C.

The conversion was 4.5 %, and the selectivities of 3FCL, 3FH, R-143a and R-123a were 71.8 %, 19.3 %, 2.6 % and 2.3 %, respectively.

Comparative Example 3

In the same manner as in Example 18 but using no thallium, the reaction was carried out.

The conversion was 12.5 %, and the selectivities of 3FCL, 3FH, R-143, R-141a, R-123a and ethane were 26.7 %, 3.7 %, 22.0 %, 21.8 %, 9.0 % and 6.6 %, respectively.

Example 19

In a glass tube reactor having an inner diameter of 10 mm, Catalyst D (1 g) comprising ruthenium as the base metal and modified with HgCl$_2$in a molar ratio of 1:1 (1 g) was charged, and through the reactor tube, a mixture of R-113 and hydrogen in a molar ratio of 1:2 was flowed at a total flow rate of 30 ml/min. at 200°C.

The conversion was 18.7 %, and the selectivities of 3FCL, 3FH and R-1132a (CF$_2$CH$_2$) were 81.5 %, 12.4 % and 1.2 %, respectively.

General procedures for supporting an additive metal on a carrier (II)

In water (30 ml), TlNO$_3$ was dissolved. Into this solution there were added formalin (0.2 g) and a pellet form palladium catalyst of 3.2 mm in diameter and 3.2 mm in height comprising 0.5 % of palladium carried on Al$_2$O$_3$ (5 g) (Catalyst A'); a catalyst prepared by pulverizing said pellet form palladium catalyst to 60 mesh or lower (4 g) (Catalyst B'); a powdery catalyst comprising 2 % of palladium carried on activated carbon (1.5 g) (Catalyst C'); or a powdery catalyst comprising 5 % of palladium or ruthenium carried on Al$_2$O$_3$ (4 g) (Catalyst D'). All catalysts are commercially available from N. E. CHEMICAL CATALYST. Then, the mixture was aged at 50°C for 2 to 3 hours. The amount of TlNO$_3$ was adjusted to obtain the intended molar ratio of Tl to the basic metal.

Then, water was removed by a rotary evaporator and the residue was dried in air at 100°C for 12 hours.

Each of the catalysts prepared from Catalysts A', B', C' and D' was pretreated in a hydrogen stream at a temperature of 300 to 400°C for 2 hours.

Example 20

The Tl/Pd molar ratio was 4:1.

In a Hastelloy C made reactor tube having an inner diameter of 20 mm, Catalyst A' (4 g) modified with TlNO$_3$ as above was charged, and through the reactor tube, a mixture of R-113 and hydrogen in a molar ratio of 1:2 was flowed at a total flow rate of 21 ml/min. at 200°C.

The conversion was 7.3 %, and the selectivities of 3FCL and R-123a were 97.2 % and 2.8 %, respectively.

## Example 21

In the same manner as in Example 20 but keeping the reaction temperature at 250°C, the reaction was carried out.

The conversion was 8.5 %, and the selectivities of 3FCL and R-123a were 98.2 % and 1.8 %, respectively.

## Example 22

In the same manner as in Example 20 but keeping the reaction temperature at 280°C, the reaction was carried out.

After one hour of the reaction, the conversion was 19.5 %, and the selectivities of 3FCL, 3FH and R-123a were 96.1 %, 2.2 % and 1.7 %, respectively. After five hours of the reaction, the conversion was 19.6 %, and the selectivities of 3FCL, 3FH and R-123a were 96.7 %, 1.9 % and 1.4 %, respectively.

## Example 23

The Tl/Pd molar ratio was 4:1.

In a stainless steel tube reactor having an inner diameter of 7.2 mm, Catalyst B′ (1.3 g) modified with TlNO$_3$ as above was charged, and through the reactor tube, a mixture of R-113 and hydrogen in a molar ratio of 1:2 was flowed at a total flow rate of 60 ml/min. at 250°C.

The conversion was 13.7 %, and the selectivities of 3FCL and 3FH were 96.4 % and 3.6 %, respectively.

## Example 24

In the same manner as in Example 23 but flowing the mixture of R-113 and hydrogen at a total flow rate of 27 ml/min., the reaction was carried out.

The conversion was 14.2 %, and the selectivities of 3FCL, 3FH and R-123a were 95.9 %, 3.5 % and 0.6 %, respectively.

## Example 25

In the same manner as in Example 24 but using Catalyst D′ modified with TlNO$_3$ in the Tl/Pd molar ratio of 1:1 (1.3 g), the reaction was carried out.

The conversion was 29.9 %, and the selectivities of 3FCL, 3FH and R-123a were 98.2 %, 1.3 % and 1.3 %, respectively.

## Example 26

In the same manner as in Example 25 but adjusting the Tl/Pd molar ratio to 2:1, the reaction was carried out.

The conversion was 22.4 %, and the selectivities of 3FCL, 3FH and R-123a were 97.7 %, 1.7 % and 0.6 %, respectively.

## Example 27

In the same manner as in Example 26 but keeping the reaction temperature at 340°C, the reaction was carried out.

The conversion was 46.5 %, and the selectivities of 3FCL, 3FH and R-123a were 95.7 %, 3.9 % and 0.5 %, respectively.

## Example 28

In the same manner as in Example 25 but using Catalyst C′ (5 g) modified with the said amount of TlNO$_3$, the reaction was carried out.

The conversion was 93.9 %, and the selectivities of 3FCL, 3FH and R-123a were 95.8 %, 4.0 % and 0.1 %, respectively.

## Example 29

In the same manner as in Example 25 but using a mixture of R-113 and hydrogen in a molar ratio of 2:1, the reaction was carried out.

The conversion was 19.9 %, and the selectivities of 3FCL, 3FH and R-123a were 97.8 %, 1.3 % and 0.9 %, respectively.

## Example 30

In the same manner as in Example 25 but using a mixture of R-113 and hydrogen in a molar ratio of 1:2.9, the reaction was carried out.

The conversion was 34.2 %, and the selectivities of 3FCL, 3FH and R-123a were 97.8 %, 1.3 % and 0.6 %, respectively.

## Example 31

In the same manner as in Example 23 but using a Catalyst C′ (0.6 g) modified with the said amount of TlNO$_3$, the reaction was carried out.

The conversion was 17.1 %, and the selectivities of 3FCL and 3FH were 92.8 % and 7.2 %, respectively.

## Example 32

In the same manner as in Example 25 but adjusting the Tl/Pd ratio to 0.5:1, the reaction was carried out.

The conversion was 57.2 %, and the selectivities of 3FH, 3FCL, R-123a, R-134a, R-141a, R-160 and R-142 were 86.7 %, 5.9 %, 3.4 %, 1.3 %, 1.2 %, 0.5 % and 0.5 %, respectively.

Comparative Example 4

In the same manner as in Example 23 without modification by thallium, the reaction was carried out.

The conversion was 64.3 %, and the selectivities of R-143, R-141a, 3FH, R-123a, R-142 and R-160 were 40.2 %, 18.7 %, 23.0 %, 7.0 %, 6.0 % and 4.6 %, respectively.

Comparative Example 5

In the same manner as in Example 31 without modification by thallium, the reaction was carried out.

The conversion was 66.6 %, and the selectivities of R-143, 3FH, R-123a, R-141a, R-152a, R-160 and R-142 were 40.2 %, 25.9 %, 13.2 %, 10.2 %, 6.0 %, 2.8 % and 1.7 %, respectively.

Example 33

The Tl/Ru molar ratio was 2:1.

In a glass tube reactor having an inner diameter of 10 mm, Catalyst D' (1 g) comprising ruthenium as the base metal and modified with TlNO$_3$ as above was charged, and through the reactor tube, a mixture of R-113 and hydrogen in a molar ratio of 3:4 was flowed at a total flow rate of 35 ml/min. at 200°C.

The conversion was 10.4 %, and the selectivities of 3FCL and R-123a were 98.8 % and 1.2 %, respectively.

Comparative Example 6

In the same manner as in Example 33 without modification by thallium, the reaction was carried out.

The conversion was 64 %, and the selectivities of 3FCL, R-123a, 3FH and R-1132a were 66.7 %, 28.6 %, 4.2 % and 1.8 %, respectively.

Claims

1.  A process for preparing 1-chloro-1,2,2-trifluoroethylene or 1,2,2-trifluoroethylene comprising reacting 1,1,2-trichloro-1,2,2-trifluoroethane and hydrogen in the presence of a catalyst which comprises at least one basic metal selected from the group consisting of palladium, rhodium and ruthenium and at least one additive metal selected from the group consisting of mercury, lead, cadmium, tin, indium, copper, bismuth, thallium and silver and a carrier selected from the group consisting of Al$_2$O$_3$, SiO$_2$ and activated carbon.

2.  The process according to claim 1, wherein an amount of said metal selected from the group consisting of palladium, rhodium and ruthenium supported on the carrier is from 0.1 to 10 % by weight.

3.  The process according to claim 2, wherein said amount is from 0.5 to 5 % by weight.

4.  The process according to claim 1, wherein a molar ratio of hydrogen to 1,1,2-trichloro-1,2,2-trifluoroethane is from 0.5 to 4.0.

5.  The process according to claim 4, wherein said ratio is from 0.5 to 3.5.

6.  The process according to claim 1, wherein a molar ratio of said additive metal to said basic metal is from 0.01:1 to 10:1.

7.  The process according to claim 6, wherein said molar ratio of said additive metal to said basic metal is from 0.2:1 to 4:1.

Patentansprüche

1.  Verfahren zur Herstellung von 1-Chlor-1,2,2-trifluorethylen oder 1,2,2-Trifluorethylen, wobei man 1,1,2-Trichlor-1,2,2-trifluorethan und Wasserstoff in der Gegenwart eines Katalysators zur Reaktion bringt, welcher mindestens 1 Basis-Metall, ausgewählt aus der Gruppe, bestehend aus Palladium, Rhodium und Ruthenium, und mindestens 1 Additiv-Metall, ausgewählt aus der Gruppe, bestehend aus Quecksilber, Blei, Cadmium, Zinn, Indium, Kupfer, Wismut, Thallium und Silber, sowie einen Träger, ausgewählt aus der Grupe, bestehend aus Al$_2$O$_3$, SiO$_2$ und aktiviertem Kohlenstoff, enthält.

2.  Verfahren gemäß Anspruch 1, worin der Mengenbereich des genannten Metalls, ausgewählt aus der Gruppe, bestehend aus Palladium, Rhodium und Ruthenium, welche auf den Träger aufgebracht sind, 0,1 bis 10 Gew.% beträgt.

3.  Verfahren gemäß Anspruch 2, worin die genannte Menge 0,5 bis 5 Gew.% beträgt.

4.  Verfahren gemäß Anspruch 1, worin das molare Verhältnis von Wasserstoff zu 1,1,2-Trichlor-1,2,2-trifluorethan 0,5 bis 4,0 beträgt.

5.  Verfahren gemäß Anspruch 4, worin das genannte Verhältnis 0,5 bis 3,5 beträgt.

6.  Verfahren gemäß Anspruch 1, worin das molare Verhältnis des genannten Additiv-Metalls zum genannten Basis-Metall 0,01:1 bis 10:1 beträgt.

7. Verfahren gemäß Anspruch 6, worin das genannte molare Verhältnis des genannten Additiv-Metalls zum genannten Basis-Metall 0,2:1 bis 4:1 beträgt.

**Revendications**

1. Procédé de préparation du 1-chloro-1,2,2-trifluoroéthylène ou du 1,2,2-trifluoroéthylène qui comprend de faire réagir du 1,1,2-trichloro-1,2,2-trifluoroéthane et de l'hydrogène en présence d'un catalyseur qui comprend au moins un métal de base choisi dans le groupe constitué du palladium, du rhodium et du ruthénium et au moins un métal supplémentaire choisi dans le groupe constitué du mercure, du plomb, du cadmium, de l'étain, de l'indium, du cuivre, du bismuth, du thallium et de l'argent et un support choisi dans le groupe constitué de $Al_2O_3$, $SiO_2$ et du charbon actif.

2. Procédé selon la revendication 1, dans lequel la quantité dudit métal choisi dans le groupe constitué du palladium, du rhodium et du ruthénium porté par le support est de 0,1 à 10 % en poids.

3. Procédé selon la revendication 2, dans lequel ladite quantité est comprise entre 0,5 et 5 % en poids.

4. Procédé selon la revendication 1, dans lequel la proportion molaire de l'hydrogène au 1,1,2-trichloro-1,2,2-trifluoroéthane est comprise entre 0,5 et 4,0.

5. Procédé selon la revendication 4, dans lequel ladite proportion est comprise entre 0,5 et 3,5.

6. Procédé selon la revendication 1, dans lequel la proportion molaire dudit métal supplémentaire audit métal de base est comprise entre 0,01:1 et 10:1.

7. Procédé selon la revendication 6, dans lequel ladite proportion molaire dudit métal supplémentaire audit métal de base est comprise entre 0,2:1 et 4:1.